# FASCICULE DE BREVET EUROPEEN

(11) **EP 4 308 215 B1**
(45) Date de publication et mention de la délivrance du brevet: **24.06.2026**
(21) Numéro de dépôt: 22714448.2
(22) Date de dépôt: 17.03.2022
(51) Int. Cl.: A61M 60/191, A61M 60/289, A61M 60/465, A61M 60/839, A61M 60/873, A61M 60/863, A61M 60/508, A61M 60/47, A61M 60/523, A61M 60/515, A61M 60/531, A61M 60/562

(54) **DISPOSITIF D'ASSISTANCE PAR COMPRESSION CARDIAQUE DIRECTE**
HILFSVORRICHTUNG DURCH DIREKTE HERZKOMPRESSION
DEVICE FOR ASSISTANCE BY DIRECT CARDIAC COMPRESSION

(30) Priorité: 17.03.2021 FR 2102652
(43) Date de publication de la demande: 24.01.2024
(73) Titulaire: Université de Lorraine, 54000 Nancy (FR); Velvet Innovative Technologies, 54850 Messein (FR); Ecole Nationale Supérieure d'Arts et Métiers (ENSAM), 75013 Paris (FR); INSTITUT NATIONAL DE LA SANTE ET DE LA RECHERCHE MEDICALE - INSERM, 75013 Paris (FR); Centre Hospitalier Régional et Universitaire de Nancy, 54500 Vandoeuvre-les-Nancy (FR)
(72) Inventeur: TRAN, Nguyen, 54280 Seichamps (FR); CHALON, Antoine, 54000 Nancy (FR); MAUREIRA, Juan-Pablo, 54710 Ludres (FR); FAVRE, Julien, 42100 Saint-Etienne (FR); PIOTROWSKI, Boris, 54610 Chenicourt (FR)
(74) Mandataire: IPAZ
(86) Numéro de dépôt international: PCT/EP2022/056918
(87) Numéro de publication internationale: WO 2022/194981

(56) Documents cités:
- US-A- 5 098 369
- US-A- 5 571 074
- US-B1- 6 238 334
- US-B1- 6 464 655

## Description

La présente invention concerne un dispositif d'assistance cardiaque par compression directe (soit Direct Cardiac Compression en Anglais), destiné au traitement de l'insuffisance cardiaque.

Plus spécifiquement, l'invention se rapporte à un dispositif implantable d'assistance péricardique fixé avantageusement à l'apex du cœur et capable d'exercer une pression localisée à des endroits déterminés de la paroi externe du cœur afin d'améliorer la fonction cardiaque. Ce dispositif peut fonctionner de façon synchrone avec le rythme natif du patient ou bien selon un rythme imposé par un dispositif de stimulation tel qu'un pacemaker implanté au préalable ou encore selon un rythme imposé par une unité de contrôle.

Le dispositif est alimenté par un stockage d'énergie, implanté selon un mode préférentiel dans le corps du patient, et qui est préférablement conçu pour être rechargé de façon transcutanée.

Les dispositifs d'assistance ventriculaire employés cliniquement pour aider les cœurs défaillants nécessitent de manière classique un contact direct entre lesdits dispositifs et le sang du patient.

Les risques d'épisodes thromboemboliques, l'hémolyse, les réactions immunitaires et les infections contribuent de manière significative à la morbidité et à la mortalité de ces dispositifs.

C'est pourquoi, la compression cardiaque directe (DCC), qui consiste à exercer une pression sur la surface péricardique du cœur, pourrait fournir un soutien ventriculaire et éviter les interactions entre le sang et un corps étranger.

En 1965, Georges Anstadt a développé un dispositif de compression péricardique destiné à la réanimation cardio-pulmonaire (RCP). Ce dispositif, connu sous le nom de cupule d'Anstadt, est une cupule de forme elliptique qui s'adapte sur les chambres ventriculaires droite et gauche. Il est doté d'une coque extérieure semi-rigide et d'un diaphragme intérieur gonflable qui fournit des forces de compression au cœur.

Plus tardivement, la société Cardio Technologies, Inc a développé le système CardioSupport ^{®} décrit dans le document EP1030701 A1 qui a pour objet un brassard ventriculaire conçu pour aider un cœur à pomper le sang en appliquant une pression uniforme sur une partie majoritaire d'une surface ventriculaire extérieure du cœur. Le brassard ventriculaire comprend une coque externe, une vessie interne gonflable et un ensemble de fixation (non représenté). La vessie possède une ouverture pour la communication avec une source de fluide sous pression de sorte que la vessie est gonflée cycliquement et dégonflée à une vitesse prédéterminée pour aider les ventricules du cœur à se contracter correctement.

D'autres travaux décrits dans le document US6464655 A1 ont porté sur un dispositif doté de plusieurs doigts pour assister sélectivement les ventricules, et en particulier le ventricule gauche, d'un cœur faible pour exercer une pression péricardique et pomper le sang d'un ou plusieurs côtés, en synchronisation avec la contraction systolique naturelle du ventricule. Ce dispositif, implantable, comprend un microprocesseur pour superviser la pluralité des doigts qui se contractent une fois que le solénoïde est alimenté électriquement et libèrent rapidement le cœur lorsque le solénoïde n'est pas alimenté. Un autre dispositif par compression cardiaque directe, comprenant plusieurs doigts dotés de coussinets de contact remplis de gel, est décrit dans le document US5098369.

Toutefois, il s'est avéré nécessaire d'améliorer les dispositifs existants en proposant une solution dans laquelle la pression exercée sur l'épicarde par les dispositifs d'assistance cardiaque directe, puisse être très précisément contrôlée au moins en termes de fréquence, d'intensité et de localisation.

C'est pourquoi, l'invention porte sur un dispositif implantable d'assistance cardiaque par compression directe caractérisé en ce qu'il comporte :
- une base formant un réceptacle apte à accueillir la partie inférieure d'un cœur,
- un support pour fixer la partie inférieure du cœur à la base, ledit support comprenant un bras de fixation et des attaches à la base, permettant d'ajuster la position dudit support sur ladite base, le support comprenant une zone d'attache conformée pour un anneau de suture apical, ledit dispositif étant conformé pour être fixé par suture entre l'apex du cœur et le support de fixation du cœur.
- au moins un doigt s'étendant longitudinalement entre une première extrémité libre supérieure et une seconde extrémité inférieure recourbée à l'intérieur de la base, ledit doigt étant monté sur ladite base selon une première liaison pivot,
- une interface de contact montée de façon réglable et amovible sur ladite extrémité libre de manière à ce que ladite interface puisse exercer une pression ajustable sur une zone localisée de l'épicarde lorsque ledit doigt est animé d'un mouvement de bascule autour de ladite liaison pivot,
- des moyens pour actionner le mouvement de bascule dudit doigt autour de ladite liaison pivot,
- une unité de pilotage desdits moyens d'actionnement.

On entend par « épicarde », la couche interne du feuillet interne (feuillet viscéral) du péricarde. Il constitue la séreuse de ce feuillet et s'étend sur toute la surface externe du cœur et la partie proximale des gros vaisseaux.

Des caractéristiques optionnelles de l'invention, complémentaires ou de substitution sont énoncées ci-après.

Selon certaines caractéristiques, les moyens pour actionner le mouvement de bascule dudit au moins un doigt autour de ladite liaison pivot, peuvent comporter une bielle et un organe coulissant dont l'une des extrémités dudit organe est en seconde liaison pivot avec ladite bielle, elle-même en troisième liaison pivot avec la seconde extrémité inférieure dudit doigt, l'autre extrémité dudit organe coulissant étant reliée au moyen d'un câble rigide ou semi rigide à un actionneur linéaire appartenant à l'unité de pilotage.

Selon un premier mode de réalisation, le dispositif peut comporter six doigts répartis de manière à ce que les six premières extrémités supérieures forment les sommet d'un hexagone, chaque seconde extrémité inférieure des six doigts étant en liaison pivot avec une bielle, elle-même en liaison pivot avec une extrémité d'un organe coulissant, l'autre extrémité dudit organe coulissant étant reliée au moyen d'un câble rigide ou semi rigide à un actionneur linéaire appartenant à l'unité de pilotage, de sorte que les doigts sont actionnés de manière indépendante.

Selon un second mode de réalisation, le dispositif peut comporter une paire de doigts disposés de manière opposée, chaque seconde extrémité inférieure des doigts étant en liaison pivot avec une bielle, elle-même en liaison pivot avec une extrémité d'un organe coulissant commun à chaque doigt, l'autre extrémité dudit organe coulissant commun étant reliée au moyen d'un seul câble rigide ou semi rigide à un actionneur linéaire appartenant à l'unité de pilotage, de sorte que les doigts sont actionnés de manière identique.

Selon d'autres caractéristiques, l'unité de pilotage peut comprendre une unité de stockage électrique pour alimenter le ou les actionneur(s) linéaire(s) et une antenne pour permettre le rechargement par voie transcutanée de ladite unité de stockage électrique.

Selon d'autres caractéristiques encore, l'unité de pilotage peut comprendre un calculateur pour piloter l'alimentation du ou des actionneur(s) linéaire(s).

Selon d'autres caractéristiques, le support pour fixer la partie inférieure du cœur à la base peut comprendre une zone d'attache de l'anneau de suture apical.

Selon d'autres caractéristiques, le support pour fixer la partie inférieure du cœur à la base peut comprendre au moins un capteur pour mesurer la pression intracardiaque.

Selon d'autres caractéristiques, le dispositif comporte des capteurs de type débit aptes à être installés sur les vaisseaux émergent du cœur, tels que l'aorte, pour permettre un rétrocontrôle dudit dispositif.

Selon d'autres caractéristiques, chaque interface de contact peut être fixée sur la première extrémité libre du doigt au moyen d'une attache coopérant avec une rainure pratiquée dans ladite extrémité de manière à ajuster la position de ladite interface le long de ladite extrémité et ainsi faire varier la localisation de la pression.

Selon d'autres caractéristiques, l'interface avec l'épicarde peut être dotée d'au moins un capteur permettant de mesurer par exemple la pression de contact.

Selon d'autres caractéristiques, le calculateur peut être connecté à un ensemble de capteurs internes ou externes au dispositif d'assistance cardiaque, afin d'adapter la fréquence et l'intensité de la pression de contact exercée par la première extrémité des doigts sur l'épicarde.

D'autres avantages et particularités de l'invention apparaîtront à la lecture de la description détaillée de mises en œuvre et de modes de réalisation nullement limitatifs, et des dessins annexés suivants :
[Fig.1] Cette figure représente une vue schématique d'un dispositif d'assistance cardiaque implanté sur un patient selon l'invention.
[Fig.2] Cette figure représente en détail une partie du dispositif d'assistance cardiaque selon un mode de réalisation de l'invention.
[Fig.3] Cette figure représente en détail une partie du dispositif d'assistance cardiaque selon un autre mode de réalisation de l'invention.
[Fig.4] Cette figure représente un schéma global du dispositif d'assistance cardiaque selon un mode de réalisation de l'invention.

Les modes de réalisation décrits ci-après étant nullement limitatifs, on pourra notamment considérer des variantes de l'invention ne comprenant qu'une sélection de caractéristiques décrites, isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

Le dispositif selon l'invention est destiné à l'implantation chez des patients souffrant d'insuffisance cardiaque terminale et ce notamment dans les cas où d'autres thérapies ne peuvent être appliquées.

L'invention peut également se substituer aux solutions thérapeutiques existantes si elle se révèle plus adaptée et fait la preuve d'un bénéfice accru pour le patient.

Le dispositif selon l'invention est destiné à l'implantation chez des patients souffrant d'insuffisance cardiaque terminale et ce notamment dans les cas où d'autres thérapies ne peuvent être appliquées.

L'invention peut également se substituer aux solutions thérapeutiques existantes si elle se révèle plus adaptée et fait la preuve d'un bénéfice accru pour le patient.

Le dispositif d'assistance cardiaque selon l'invention génère une déformation mécanique de la paroi externe de l'organe de façon synchrone et asservie aux besoins de l'organe défaillant.

Le dispositif permet de compenser l'insuffisance de la fonction de pompe cardiaque sans être en contact direct avec le sang circulant du patient et sans créer de rupture de la barrière de la peau, source classique d'infection.

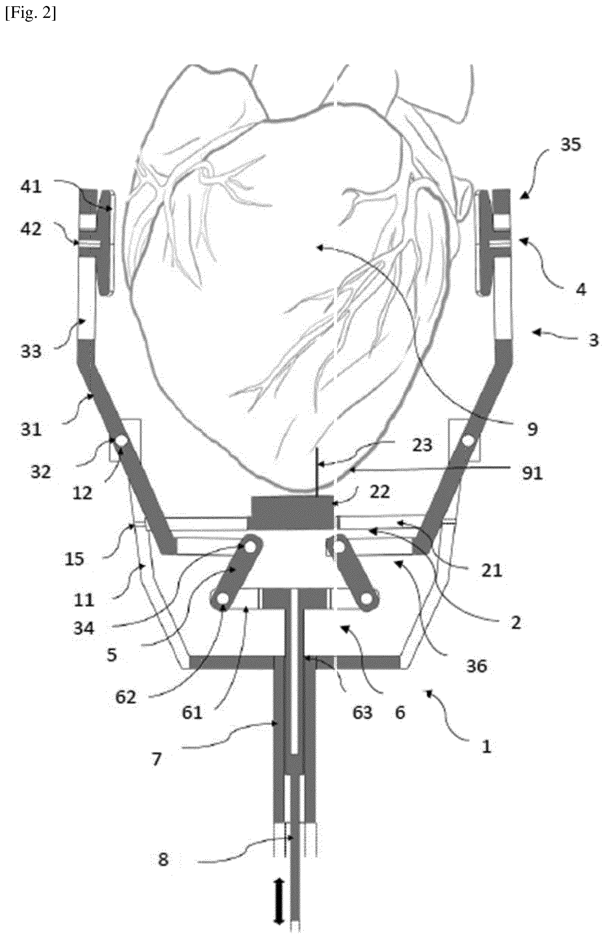

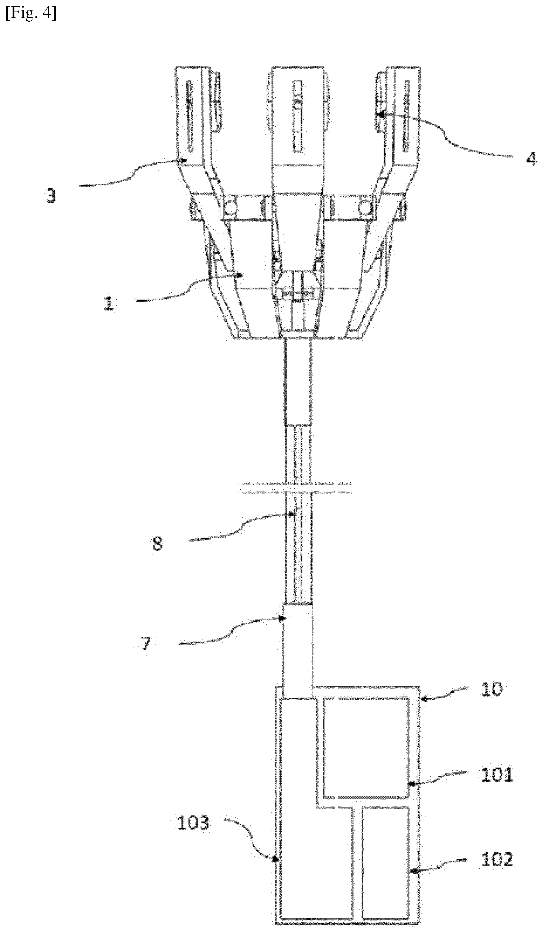

Le dispositif d'assistance cardiaque selon l'invention et tel que représenté sur les figures 2, 3, 4 comporte à minima :
- une base 1 formant un réceptacle apte à accueillir la partie inférieure d'un cœur 9,
- un support 2 pour fixer la partie inférieure du cœur à la base, ledit support comprenant un bras de fixation 21 et des attaches 15 à la base 1, permettant d'ajuster la position dudit support sur ladite base,
- au moins un doigt 3 s'étendant longitudinalement entre une première extrémité 35 libre supérieure et une seconde extrémité 36 inférieure recourbée à l'intérieur de la base, ledit doigt étant monté sur ladite base selon une première liaison pivot 12,
- une interface de contact 4 montée de façon réglable et amovible sur ladite extrémité libre de manière à ce que ladite interface puisse exercer une pression ajustable sur une zone localisée de l'épicarde lorsque ledit doigt est animé d'un mouvement de bascule autour de ladite liaison pivot,
- des moyens 5, 6 pour actionner le mouvement de bascule dudit doigt autour de ladite liaison pivot,
- une unité de pilotage 10 desdits moyens d'actionnement 5, 6.

Bien entendu, le dispositif selon l'invention peut comporter un ou plusieurs doigts.

Le fait que la seconde extrémité 36 inférieure du au moins un doigt soit recourbée à l'intérieur de la base, permet d'épouser la forme du cœur et de rendre ainsi le dispositif moins encombrant.

La première liaison pivot 12 du au moins un doigt, qui est réalisée par exemple au moyen d'un perçage 32 dans le corps 31 dudit doigt, n'est pas disposée en l'extrémité inférieure dudit doigt, mais plutôt de préférence quelque part entre le milieu dudit doigt et son extrémité inférieure.

Ainsi, par effet de levier, le mouvement de bascule consomme une énergie moindre.

Dans tous les cas, c'est le réglage de la position des interfaces de contact au niveau des extrémités des doigts qui permet d'ajuster la pression de contact sur l'épicarde ainsi que la zone précise à stimuler.

Le dispositif est donc prévu pour exercer une pression sur le cœur (systole) et pour rester ouvert et éloigné du cœur de manière inactive si les moyens d'actionnement ne sont plus pas alimentés.

Cette désactivation des moyens d'actionnement permet au cœur de se dilater librement (diastole) après la compression pour permettre au sang de retourner naturellement et librement vers les ventricules cardiaques et le réseau vasculaire coronaire (artères et veines).

La pression externe exercée par le myocarde pendant la systole ventriculaire comprime le muscle cardiaque (myocarde) et diminue le flux sanguin vers les artères coronaires qui alimentent le cœur même si la pression aortique est augmentée. Ainsi, plus de 70% du flux artériel coronaire se produit pendant la diastole.

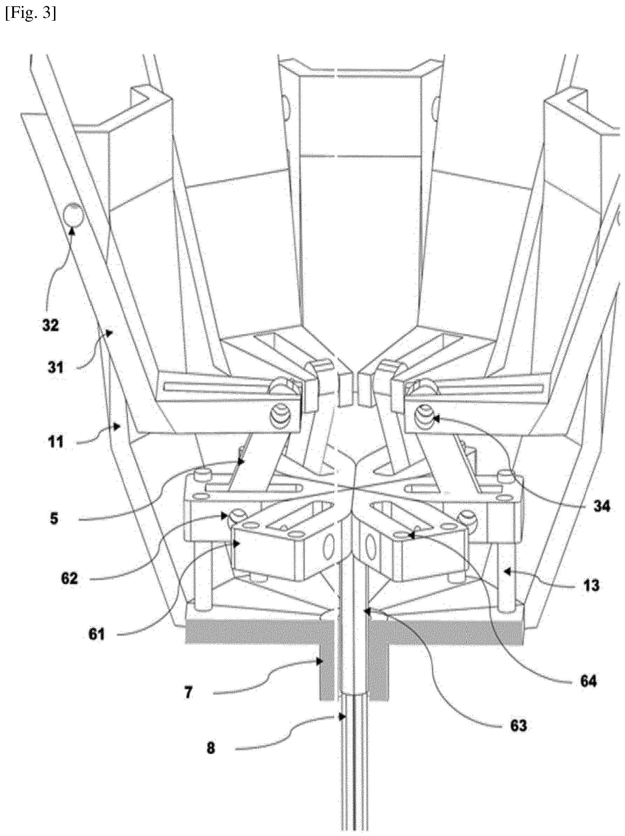

Selon des modes de réalisation préférés représentés sur les figures 2 et 3, les moyens 5, 6 pour actionner le mouvement de bascule d'un doigt autour de ladite liaison pivot, comportent une bielle 5 et un organe coulissant 6 dont l'une des extrémités 61 est en liaison pivot 62 avec ladite bielle, elle-même en liaison pivot 34 avec la seconde extrémité 36 du doigt 3.

L'autre extrémité de l'organe coulissant 6 est alors reliée au moyen d'au moins un câble rigide ou semi rigide 8 à un actionneur linéaire 103 de l'unité de pilotage 10.

Ainsi, le câble 8, logé dans une gaine 7 permet de déporter la partie moteur, la source d'énergie et l'unité électronique à distance de la cage thoracique où l'espace est très limité.

Le mouvement de translation généré par le ou les actionneur(s) linéaire(s) 103 est transmis par un ou plusieurs câbles rigides ou semi rigides 8 contenus dans la gaine 7 jusqu'à la partie du dispositif autour du cœur. La translation de l'organe coulissant 6 dans la base 1 est transmise au doigt 3 par l'intermédiaire de la bielle 5 au niveau de la liaison pivot 62 entre l'organe coulissant 6 et la bielle 5, puis au niveau de la liaison pivot 34 entre la bielle 5 et la seconde extrémité 36 du doigt 3.

Ce mouvement engendre un mouvement de bascule, soit une rotation du doigt autour de sa liaison pivot 12 avec la base 1, avec pour conséquence une réduction concentrique de l'écart entre l'extrémité des doigts.

Selon le premier mode de réalisation représenté en [Fig.2], le dispositif comporte une paire de doigts disposés de manière opposée.

Chaque seconde extrémité inférieure 36 des deux doigts 3 est en liaison pivot 34 avec une bielle 5 propre à chacun des doigts, elle-même en liaison pivot 62 avec une extrémité 61 d'un organe 6 coulissant et commun à chaque doigt.

L'autre extrémité de l'organe coulissant commun 6 est reliée au moyen d'un seul câble rigide ou semi rigide 8 à un actionneur linéaire 103 appartenant à l'unité de pilotage 10, de sorte que les doigts sont actionnés de manière identique.

Selon le second mode de réalisation représenté en [Fig.3], le dispositif comporte six doigts répartis de manière à ce que les six extrémités supérieures forment les sommets d'un hexagone.

Chaque seconde extrémité inférieure 36 des six doigts 3 est en liaison pivot 34 avec une bielle 5 propre à chaque doigt, elle-même en liaison pivot 62 avec une extrémité 61 d'un organe coulissant 6, propre à chaque doigt. Le guidage du coulissement de chaque organe 6 s'effectue selon au moins un axe 13 au travers d'un orifice 64 pratiqué au voisinage de l'extrémité 61.

L'autre extrémité de chaque organe coulissant 6 est reliée au moyen d'un câble rigide ou semi rigide 8 à un actionneur linéaire 103 appartenant à l'unité de pilotage 10, de sorte que les doigts sont actionnés de manière indépendante.

Une seule et même gaine 7 renferme alors l'ensemble des six câbles 8.

Selon les besoins, il est possible de regrouper les organes coulissants entre eux, de les rendre solidaires ou bien qu'ils partagent un même câble d'actionnement ou le même actionneur linéaire.

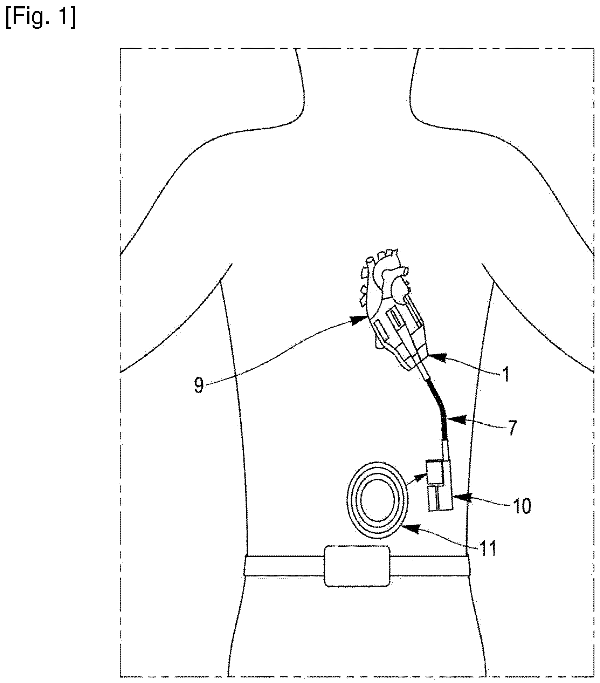

Avantageusement et tel que représenté sur la [Fig.1], l'unité de pilotage 10 comprend une unité de stockage électrique 101 pour alimenter l'actionneur linéaire 103 et une antenne 11 pour permettre le rechargement par voie transcutanée de ladite unité de stockage électrique.

Bien entendu, d'autres configurations d'alimentation électrique peuvent être envisagées, avec par exemple, le branchement de l'unité de pilotage en externe.

L'emplacement préférentiel pour cette unité de pilotage 10 est l'abdomen, avec la possibilité de recharger la batterie intégrée par induction transcutanée.

De manière préférentielle, le support 2 pour fixer la partie inférieure du cœur à la base comprend une zone d'attache 22 de l'anneau de suture apical.

En d'autres termes, le dispositif est fixé idéalement par suture entre l'apex 91 du cœur et le support de fixation du cœur 2. Ce support 2 est monté sur la base 1 par des attaches traversantes 15 au niveau des bras de fixation 21.

L'ensemble formé par la base 1 et les doigts 3 est ainsi solidarisé au cœur au niveau de l'apex, et épouse au plus près sa partie inférieure.

L'anneau de suture apical, qui est connu de l'homme du métier, est dans le cas présent un dispositif annexe à l'assistance cardiaque décrite dans l'invention, qui joue le rôle d'interface entre l'organe et le dispositif. En effet, une des conditions clés pour assurer l'efficacité de la compression du cœur par l'invention est de prévenir l'échappement de l'organe ainsi qu'une déformation externe aux endroits d'intérêt et non ailleurs.

La faisabilité de la fixation d'un dispositif d'assistance cardiaque dans la zone apicale du cœur a déjà été étudiée dans la publication « Chalon A, Favre J, Piotrowski B, Landmann V, Grandmougin D, Maureira JP, Laheurte P, Tran N. Contribution of computational model for assessment of heart tissue local stress caused by suture in LVAD implantation. J Mech Behav Biomed Mater. 2018 Jun;82:291-298. doi: 10.1016/j.jmbbm.2018.03.032. Epub 2018 Mar 28. PMID: 29649657.»

L'anneau de suture apical est généralement constitué de deux parties.

La partie externe est réalisée, selon une première méthode de fabrication, en Téflon^{®} de grade médical (PTFE), ou bien en Dacron^{®} ou bien dans un autre tissu synthétique biocompatible et non résorbable. Selon une seconde méthode de fabrication de cette partie externe, il est possible d'utiliser du tissu péricardique d'une autre espèce (bovin, ovin ou autre) traité afin d'être toléré par l'organisme receveur. Cette partie externe (anneau externe) est fixée à l'anneau interne par sertissage, collage et/ou suture afin que ces deux éléments restent solidaires dans le temps.

L'anneau interne est réalisé en matière biocompatible, telle que, mais non limitée à, l'inox dopé à l'azote le titane et alliages de titane (Ti-Nb par exemple) ou un polymère (PEEK, UHMWPE ou PP par exemple). Cette section comporte un segment fixe assurant la liaison avec l'anneau externe et un segment de bride circulaire, permettant la connexion ferme avec le dispositif d'assistance cardiaque. Une vis ou un levier de réglage permettent de serrer cette bride circulaire sur la zone d'attache du dispositif d'assistance cardiaque selon l'invention. Le serrage et / ou le verrouillage permettent d'empêcher la rotation du dispositif par rapport à l'organe.

Selon un autre mode de réalisation, il est possible de ménager une bride non circulaire (hexagonale par exemple) permettant le verrouillage des éléments entre eux et limitant les risques de rotation de l'anneau de suture par rapport au dispositif d'assistance cardiaque.

Ainsi, la partie externe est suturée à l'apex du cœur et la partie interne est verrouillée au dispositif d'assistance cardiaque pour assurer un maintien en place de l'invention et un bon fonctionnement de cette dernière.

Concernant la zone d'attache 22 de l'anneau apical sur le dispositif, elle émerge du support 2, connectée au travers des attaches 15 au bâti 1. Cette zone d'attache peut être cylindrique ou d'une forme complémentaire à la bride précédemment décrite afin de prévenir toute rotation non désirée des éléments les uns par rapport aux autres. Cet élément d'attache est creux afin d'accueillir des tubulures et/ou des capteurs de pression. Un fenêtrage dans une des émergences de la base 1) permet de faire traverser tubulures et/ou câbles nécessaires sans gêner le fonctionnement de l'invention.

Une canule, tubulure ou aiguille rigide ou non 23 émerge de la zone d'attache 22 et est dimensionnée pour que son extrémité libre s'ouvre à l'intérieur du ventricule gauche. Reliée directement à un capteur au creux de la zone d'attache 22 ou par une tubulure jusqu'à un capteur situé à distance (notamment au niveau de l'unité de pilotage 10), le capteur 23 rend possible la mesure en continu de la pression ventriculaire gauche qui sera utilisée par l'unité de pilotage 10 pour adapter l'action du dispositif au besoin du patient. Dans l'un ou l'autre des cas, tubulure ou câbles courent le long du bâti 1 et suivent le trajet de la gaine 7 pour gagner l'unité de pilotage 10.

Avantageusement, le dispositif comporte des capteurs de type débit aptes à être installés sur les vaisseaux émergent du cœur, tels que l'aorte, pour permettre un rétrocontrôle dudit dispositif.

Avantageusement, l'interface de contact 4 est fixée sur la première extrémité libre 35 du doigt 3 au moyen d'une attache 42 coopérant avec une rainure 33 pratiquée dans ladite extrémité de manière à ajuster la position de ladite interface le long de ladite extrémité, et d'ajuster également sa distance vis-à-vis de l'épicarde. Le caractère amovible permet par ailleurs de pratiquer au remplacement de l'interface avant l'implantation du dispositif sur le patient.

Ces éléments d'interface 4, réglables, amovibles et sur-mesure sont donc fixés selon une attache 42 dans la rainure 33 de la partie supérieure des doigts et opèrent le contact avec l'épicarde.

Ce mouvement génère un phénomène de compression sur la surface externe du cœur qui induit une augmentation de pression dans les ventricules et favorise l'éjection du sang vers les circulations pulmonaire et systémique.

Avantageusement, ces éléments d'interface 4 avec l'épicarde peuvent être dotés d'au moins un capteur permettant de mesurer la pression de contact.

De même, le support 2 pour fixer la partie inférieure du cœur à la base peut comprendre au moins un capteur 23 pour mesurer la pression intracardiaque. Ainsi, des capteurs de pression, peuvent être installés dans le support de fixation du cœur 2 et reliés à des canules 23 placées chirurgicalement dans les ventricules.

Dès lors, il est possible de conditionner l'amplitude et/ou la force de pression des doigts en fonction des signaux recueillis par cet ensemble de capteurs, notamment pour synchroniser l'action du dispositif avec la contraction du cœur.

Pour ce faire, l'unité de pilotage 10 comporte un calculateur 102 pour piloter l'alimentation de l'actionneur linéaire 103.

Ce calculateur 102 est connecté à un ensemble de capteurs internes ou externes au dispositif d'assistance cardiaque, afin d'adapter la fréquence et l'intensité de la pression de contact exercée par la première extrémité des doigts sur l'épicarde.

L'acquisition et la gestion de ces signaux est donc effectuée dans l'unité de pilotage 10 et plus précisément par le calculateur 102.

Des capteurs de type débit peuvent aussi être installés sur des vaisseaux émergent du cœur, typiquement l'aorte, pour permettre un rétrocontrôle de l'action de l'invention.

Le retour en position inverse permet un relâchement de l'ensemble des articulations et un retour à la position « ouverte » initiale. Cette position initiale peut être calibrée pour exercer une pression minimale sur l'organe pour compenser sa dilatation pathologique.

Les matériaux qui composent l'invention et qui sont implantées dans le patient sont tous conçus avec des matériaux reconnus scientifiquement et industriellement comme biocompatibles, ou bien, le cas échéant sont isolés du patient par de tels matériaux.

Le dispositif peut être fabriqué tout ou partie par impression 3D à partir de poudre de TiNb ou de PEEK.

Des tests mécaniques sur banc d'essai et sur organe ex vivo en conditions iso-volumiques ont été réalisés afin de rendre compte des forces et pressions générées par le dispositif ainsi que de l'énergie requise pour créer ces efforts. Les résultats ont clairement montré une capacité du dispositif à restaurer les paramètres hémodynamiques même sur un cœur complètement arrêté, à savoir la pression intraven-triculaire, la pression artérielle aortique à l'identique d'un fonctionnement physiologique normale.

De plus, cela a permis de montrer que l'énergie déployée par le dispositif pour ce travail d'assistance cardiaque reste à un niveau suffisamment faible pour envisager son miniaturisation et son implantation complète.

Les premiers tests réalisés à l'aide de prototypes de l'invention décrite ont permis d'établir une première estimation des performances du dispositif.

En premier lieu, les essais sur cœur explantés en condition iso-volumique (aucune circulation de fluide, les cavités cardiaques étaient remplies d'un analogue sanguin) ont montré la possibilité d'atteindre des pressions intracardiaques allant jusqu'à 110 mm Hg, ce qui correspond à la typique de tension artérielle chez un adulte sain.

Des tests ultérieurs ont démontré la possibilité de monter au-delà.

Toutefois, ces valeurs bien trop hautes physiologiquement illustrent uniquement la réserve de force que peut développer le dispositif et ne relève pas de son usage prévu en clinique.

Le dispositif peut suivre une fréquence de contraction jusqu'à 80 battements par minute sans générer de perte de son efficacité contractile. Au-delà, il est possible d'atteindre des valeurs de 120 battements par minute mais avec une usure prématurée du dispositif, un potentiel effet délétère sur le cœur et enfin des performances largement dégradées.

Le temps nécessaire pour obtenir la force maximale du dispositif a été relevé aux environs de 100+/25 ms mais peut être grandement amélioré jusqu'à 20+/-10 ms.

La consommation énergétique a été estimée à 155 KJ pour 24 heures d'utilisation continue à un rythme de 60 battements par minute. A titre de comparaison, c'est la quantité d'énergie contenue dans une batterie d'ordinateur portable.

Vis-à-vis des autres thérapies classiques, le dispositif implantable d'assistance cardiaque par compression directe selon l'invention présente les avantages suivants :
- Technique conservative de l'organe
- Préservation de la pulsatilité du débit
- Diminution du volume de la cavité cardiaque
- Augmentation du débit cardiaque
- Absence de contact sanguin directe
- Implantation totale du dispositif (par mini-thoracotomie) et de ses annexes chez le patient (diminution des risques infectieux).

En d'autres termes, le dispositif implantable d'assistance cardiaque par compression directe selon l'invention est ajusté au plus près de la morphologie du cœur en respectant les zones saines d'appui ainsi que les zones fragiles et les zones infarcies.

A noter, les différentes caractéristiques, formes, variantes et modes de réalisation de l'invention peuvent être associés les uns avec les autres, selon diverses combinaisons dans la mesure où ils ne sont pas incompatibles ou exclusifs les uns des autres.

## Revendications

1. Dispositif implantable d'assistance cardiaque par compression directe **caractérisé en ce qu'**il comporte :
- une base (1) formant un réceptacle apte à accueillir la partie inférieure d'un cœur (9),
- un support (2) pour fixer la partie inférieure du cœur à ladite base, ledit support comprenant un bras de fixation (21) et des attaches (15) à la base (1), permettant d'ajuster la position dudit support sur ladite base, le support (2) comprenant une zone d'attache (22) conformée pour un anneau de suture apical, ledit dispositif étant conformé pour être fixé par suture entre l'apex (91) du cœur et le support de fixation du cœur.
- au moins un doigt (3) s'étendant longitudinalement entre une première extrémité (35) libre supérieure et une seconde extrémité (36) inférieure recourbée à l'intérieur de la base, ledit doigt étant monté sur ladite base selon une première liaison pivot (12)
- une interface de contact (4) montée de façon réglable et amovible sur ladite extrémité libre de manière à ce que ladite interface puisse exercer une pression ajustable sur une zone localisée de l'épicarde, lorsque ledit doigt est animé d'un mouvement de bascule autour de ladite liaison pivot
- des moyens (5, 6) pour actionner le mouvement de bascule dudit doigt autour de ladite liaison pivot,
- une unité de pilotage (10) desdits moyens d'actionnement (5, 6).

2. Dispositif implantable d'assistance cardiaque par compression directe, selon la revendication 1, **caractérisé en ce que** les moyens (5, 6) pour actionner le mouvement de bascule dudit au moins un doigt autour de ladite liaison pivot, comportent une bielle (5) et un organe coulissant (6) dont l'une des extrémités (61) est en seconde liaison pivot (62) avec ladite bielle, elle-même en troisième liaison pivot (34) avec la seconde extrémité inférieure (36) dudit doigt (3), l'autre extrémité de l'organe coulissant (6) étant reliée au moyen d'un câble rigide ou semi rigide (8) à un actionneur linéaire (103) appartenant à l'unité de pilotage (10).

3. Dispositif implantable d'assistance cardiaque par compression directe, selon l'une quelconque des revendications précédentes, **caractérisé en ce que** le dispositif comporte six doigts répartis de manière à ce que les six extrémités supérieures forment les sommet d'un hexagone, chaque seconde extrémité inférieure (36) des six doigts (3) étant en liaison pivot (34) avec une bielle (5), elle-même en liaison pivot (62) avec une extrémité (61) d'un organe coulissant (6), l'autre extrémité de l'organe coulissant (6) étant reliée au moyen d'un câble rigide ou semi rigide (8) à un actionneur linéaire (103) appartenant à l'unité de pilotage (10), de sorte que les doigts sont actionnés de manière indépendante.

4. Dispositif implantable d'assistance cardiaque par compression directe, selon l'une quelconque des revendications précédentes, **caractérisé en ce que** le dispositif comporte une paire de doigts disposés de manière opposée, chaque seconde extrémité inférieure (36) des doigts (3) étant en liaison pivot (34) avec une bielle (5), elle-même en liaison pivot (62) avec une extrémité (61) d'un organe coulissant commun (6) à chaque doigt, l'autre extrémité de l'organe coulissant commun (6) étant reliée au moyen d'un seul câble rigide ou semi rigide (8) à un actionneur linéaire commun (103) appartenant à l'unité de pilotage (10), de sorte que les doigts sont actionnés de manière identique.

5. Dispositif implantable d'assistance cardiaque par compression directe, selon la revendication précédente, **caractérisé en ce que** l'unité de pilotage (10) comprend une unité de stockage électrique (101) implantable, pour alimenter le ou les actionneur(s) linéaire(s) (103) et une antenne (11) pour permettre le rechargement par voie transcutanée de ladite unité de stockage électrique.

6. Dispositif implantable d'assistance cardiaque par compression directe, selon l'une quelconque des revendications 2 à 5, **caractérisé en ce que** l'unité de pilotage (10) comprend un calculateur (102) pour piloter l'alimentation du ou des actionneur(s) linéaire(s) (103).

7. Dispositif implantable d'assistance cardiaque par compression directe, selon l'une quelconque des revendications précédentes, **caractérisé en ce que** le support (2) pour fixer la partie inférieure du cœur à la base comprend au moins un capteur (23) pour mesurer la pression intracardiaque.

8. Dispositif implantable d'assistance cardiaque par compression directe, selon l'une quelconque des revendications précédentes, **caractérisé en ce que** le dispositif comporte des capteurs de type débit aptes à être installés sur les vaisseaux émergent du cœur, tels que l'aorte, pour permettre un rétrocontrôle dudit dispositif.

9. Dispositif implantable d'assistance cardiaque par compression directe, selon l'une quelconque des revendications précédentes, **caractérisé en ce que** l'interface de contact (4) est fixée sur la première extrémité libre (35) de son doigt (3) au moyen d'une attache (42) coopérant avec une rainure (33) pratiquée dans ladite extrémité, de manière à ajuster la position de ladite interface le long de ladite extrémité.

10. Dispositif implantable d'assistance cardiaque par compression directe, selon l'une quelconque des revendications précédentes, **caractérisé en ce que** l'interface de contact (4) de chaque doigt avec l'épicarde est dotée d'au moins un capteur permettant par exemple de mesurer la pression de contact.

11. Dispositif implantable d'assistance cardiaque par compression directe, selon la revendication 6, **caractérisé en ce que** le calculateur (102) est connecté à un ensemble de capteurs internes ou externes au dispositif d'assistance cardiaque, afin d'adapter la fréquence et l'intensité de la pression de contact exercée par la première extrémité des doigts sur l'épicarde.

## Patentansprüche

1. Implantierbare Vorrichtung zur Herzunterstützung durch direkte Kompression, **dadurch gekennzeichnet, dass** sie vorweist:
- eine Basis (1), die ein Behältnis ausbildet, das geeignet ist, um den unteren Teil eines Herzens (9) aufzunehmen,
- einen Träger (2) zum Befestigen des unteren Teils des Herzens an der Basis, der Träger umfassend einen Befestigungsarm (21) und Anbringungselemente (15) an der Basis (1), die es ermöglichen, die Position des Trägers auf der Basis einzustellen, der Träger (2) umfassend einen Anbringungsbereich (22), der für einen apikalen Nahtring angepasst ist, wobei die Vorrichtung angepasst ist, um durch Naht zwischen dem Apex (91) des Herzens und dem Träger der Herzbefestigung befestigt zu werden.
- mindestens einen Finger (3), der sich in Längsrichtung zwischen einem ersten oberen freien Ende (35) und einem zweiten unteren Ende (36) erstreckt, das im Inneren der Basis gekrümmt ist, wobei der Finger auf der Basis gemäß einer ersten Schwenkverbindung (12) montiert ist
- eine Berührungsfläche (4), die regulierbar und abnehmbar an dem freien Ende montiert ist, in der Art, dass die Fläche einen einstellbaren Druck auf einen lokalisierten Bereich des Epikards ausüben kann, wenn der Finger in eine Kippbewegung um die Schwenkverbindung angetrieben wird
- Mittel (5, 6) zum Betätigen der Kippbewegung des Fingers um die Schwenkverbindung,
- eine Steuereinheit (10) der Betätigungsmittel (5, 6).

2. Implantierbare Vorrichtung zur Herzunterstützung durch direkte Kompression nach Anspruch 1, **dadurch gekennzeichnet, dass** die Mittel (5, 6) zum Betätigen der Kippbewegung des mindestens einen Fingers um die Schwenkverbindung eine Pleuelstange (5) und ein Gleitelement (6) vorweisen, wovon eines der Enden (61) in einer zweiten Schwenkverbindung (62) mit der Pleuelstange steht, die selbst in einer dritten Schwenkverbindung (34) mit dem zweiten unteren Ende (36) des Fingers (3) steht, wobei das andere Ende des Gleitelements (6) mittels eines starren oder halbstarren Kabels (8) mit einem linearen Aktuator (103) verbunden ist, der zu der Steuereinheit (10) gehört.

3. Implantierbare Vorrichtung zur Herzunterstützung durch direkte Kompression nach einem der vorstehenden Ansprüche, **dadurch gekennzeichnet, dass** die Vorrichtung sechs Finger vorweist, die in der Art verteilt sind, dass die sechs oberen Enden die Scheitelpunkte eines Sechsecks ausbilden, wobei jedes zweite untere Ende (36) der sechs Finger (3) in Schwenkverbindung (34) mit einer Pleuelstange (5) steht, die ihrerseits in Schwenkverbindung (62) mit einem Ende (61) eines Gleitorgans (6) steht, wobei das andere Ende des Gleitorgans (6) mittels eines starren oder halbstarren Kabels (8) mit einem linearen Aktuator (103) verbunden ist, der zu der Steuereinheit (10) gehört, sodass die Finger unabhängig betätigt werden.

4. Implantierbare Vorrichtung zur Herzunterstützung durch direkte Kompression nach einem der vorstehenden Ansprüche, **dadurch gekennzeichnet, dass** die Vorrichtung ein Paar von gegenüberliegend angeordneten Fingern vorweist, wobei jedes zweite untere Ende (36) der Finger (3) in Schwenkverbindung (34) mit einer Pleuelstange (5) steht, die ihrerseits in Schwenkverbindung (62) mit einem Ende (61) eines gemeinsamen Gleitorgans (6) an jedem Finger steht, wobei das andere Ende des gemeinsamen Gleitorgans (6) mittels eines einzigen starren oder halbstarren Kabels (8) mit einem gemeinsamen linearen Aktuator (103) verbunden ist, der zu der Steuereinheit (10) gehört, sodass die Finger identisch betätigt werden.

5. Implantierbare Vorrichtung zur Herzunterstützung durch direkte Kompression nach dem vorstehenden Anspruch, **dadurch gekennzeichnet, dass** die Steuereinheit (10) eine implantierbare elektrische Speichereinheit (101) zum Versorgen des oder der linearen Aktuators/Aktuatoren (103) und eine Antenne (11) zum Ermöglichen des Aufladens auf transkutanem Weg der der elektrischen Speichereinheit, umfasst.

6. Implantierbare Vorrichtung zur Herzunterstützung durch direkte Kompression nach einem der Ansprüche 2 bis 5, **dadurch gekennzeichnet, dass** die Steuereinheit (10) einen Rechner (102) zum Steuern der Versorgung des oder der linearen Aktuators/Aktuatoren (103) umfasst.

7. Implantierbare Vorrichtung zur Herzunterstützung durch direkte Kompression nach einem der vorstehenden Ansprüche, **dadurch gekennzeichnet, dass** der Träger (2) zum Befestigen des unteren Teils des Herzens an der Basis mindestens einen Sensor (23) zum Messen des intrakardialen Drucks umfasst.

8. Implantierbare Vorrichtung zur Herzunterstützung durch direkte Kompression nach einem der vorstehenden Ansprüche, **dadurch gekennzeichnet, dass** die Vorrichtung Durchflusssensoren vorweist, die geeignet sind, um an den aus dem Herzen austretenden Gefäßen, wie der Aorta, zum Ermöglichen einer Rückkopplung der Vorrichtung installiert zu werden.

9. Implantierbare Vorrichtung zur Herzunterstützung durch direkte Kompression nach einem der vorstehenden Ansprüche, **dadurch gekennzeichnet, dass** die Berührungsfläche (4) an dem ersten freien Ende (35) ihres Fingers (3) mittels eines Anbringungselements (42) befestigt ist, das mit einer in dem Ende ausgeführten Nut (33) zusammenwirkt, um die Position der Fläche entlang des Endes einzustellen.

10. Implantierbare Vorrichtung zur Herzunterstützung durch direkte Kompression nach einem der vorstehenden Ansprüche, **dadurch gekennzeichnet, dass** die Berührungsfläche (4) jedes Fingers mit dem Epikard mit mindestens einem Sensor versehen ist, der zum Beispiel das Messen des Berührungsdrucks ermöglicht.

11. Implantierbare Vorrichtung zur Herzunterstützung durch direkte Kompression nach Anspruch 6, **dadurch gekennzeichnet, dass** der Rechner (102) an einen Satz von Sensoren, intern oder extern der Vorrichtung zur Herzunterstützung, angeschlossen ist, um die Frequenz und die Intensität des Berührungsdrucks anzugleichen, der durch das erste Ende der Finger auf das Epikard ausgeübt wird.

## Claims

1. Implantable device for cardiac assistance by direct compression, **characterized in that** it includes:
- a base (1) forming a receptacle capable of receiving the lower part of a heart (9),
- a support (2) for fixing the lower part of the heart to said base, said support comprising a fixing arm (21) and fasteners (15) at the base (1), making it possible to adjust the position of said support on said base, the support (2) comprising an area for fastening (22) configured to an apical suture ring, said device being configured to be fixed by suturing between the apex (91) of the heart and the fixing support of the heart (2),
- at least one finger (3) extending longitudinally between a free upper first end (35) and a curved lower second end (36) inside the base, said finger being mounted on said base by virtue of a first pivot connection (12),
- a contact interface (4) mounted adjustably and movably on said free end so that said interface can exert an adjustable pressure on a localized area of the epicardium, when said finger is moved by a tilting movement about said pivot connection,
- means (5, 6) for actuating the tilting movement of said finger about said pivot connection,
- a unit for controlling (10) said actuating means (5, 6).

2. Implantable device for cardiac assistance by direct compression, according to claim 1, **characterized in that** the means (5, 6) for actuating the tilting movement of said at least one finger about said pivot connection include a connecting rod (5) and a sliding member (6) one of the ends (61) of which is in a second pivot connection (62) with said connecting rod, itself in a third pivot connection (34) with the lower second end (36) of said finger (3), the other end of the sliding member (6) being connected by means of a rigid or semi-rigid cable (8) to a linear actuator (103) belonging to the control unit (10).

3. Implantable device for cardiac assistance by direct compression, according to any one of the preceding claims, **characterized in that** the device includes six fingers distributed so that the six upper ends form the vertices of a hexagon, each lower second end (36) of the six fingers (3) being in a pivot connection (34) with a connecting rod (5), itself in a pivot connection (62) with an end (61) of a sliding member (6), the other end of the sliding member (6) being connected by means of a rigid or semi-rigid cable (8) to a linear actuator (103) belonging to the control unit (10), so that the fingers are actuated independently.

4. Implantable device for cardiac assistance by direct compression, according to any one of the preceding claims, **characterized in that** the device includes a pair of fingers arranged opposite one another, each lower second end (36) of the fingers (3) being in a pivot connection (34) with a connecting rod (5), itself in a pivot connection (62) with an end (61) of a sliding member (6) common to each finger, the other end of the common sliding member (6) being connected by means of a single rigid or semi-rigid cable (8) to a common linear actuator (103) belonging to the control unit (10), so that the fingers are actuated identically.

5. Implantable device for cardiac assistance by direct compression, according to the preceding claim, **characterized in that** the control unit (10) comprises an implantable electrical storage unit (101) to power the linear actuator(s) (103) and an antenna (11) to allow recharging of said electrical storage unit by the transcutaneous route.

6. Implantable device for cardiac assistance by direct compression, according to any one of claims 2 to 5, **characterized in that** the control unit (10) comprises a computer (102) for controlling powering of the linear actuator(s) (103).

7. Implantable device for cardiac assistance by direct compression, according to any one of the preceding claims, **characterized in that** the support (2) for fixing the lower part of the heart to the base comprises at least one sensor (23) for measuring the intracardiac pressure.

8. Implantable device for cardiac assistance by direct compression, according to any one of the preceding claims, **characterized in that** the device includes sensors of the flow type capable of being installed on the vessels emerging from the heart, such as the aorta, to allow feedback from said device.

9. Implantable device for cardiac assistance by direct compression, according to any one of the preceding claims, **characterized in that** the contact interface (4) is fixed on the free first end (35) of its finger (3) by means of a fastener (42) cooperating with a groove (33) made in said end, so as to adjust the position of said interface along said end.

10. Implantable device for cardiac assistance by direct compression, according to any one of the preceding claims, **characterized in that** the interface for contact (4) of each finger with the epicardium is equipped with at least one sensor making it possible for example to measure the contact pressure.

11. Implantable device for cardiac assistance by direct compression, according to claim 6, **characterized in that** the computer (102) is connected to an assembly of sensors internal or external to the cardiac assistance device, in order to adapt the frequency and the intensity of the contact pressure exerted by the first end of the fingers on the epicardium.
